# EUROPEAN PATENT SPECIFICATION

(11) **EP 2 943 562 B1**
(45) Date of publication and mention of the grant of the patent: **22.06.2016**
(21) Application number: 14705052.0
(22) Date of filing: 22.01.2014
(51) Int. Cl.: C12M 1/00, C12M 1/33, B01L 3/00

(54) **A SYSTEM FOR EXTRACTION OF CELLS FROM A SAMPLE OF TISSUE**
SYSTEM ZUR EXTRAKTION VON ZELLEN AUS EINER GEWEBEPROBE
SYSTÈME D'EXTRACTION DE CELLULES DANS UN ÉCHANTILLON DE TISSU

(30) Priority: 04.03.2013 WO PCT/EP2013/054297
(43) Date of publication of application: 18.11.2015
(73) Proprietor: SWISS STEM CELL FOUNDATION, 6925 Gentilino (CH)
(72) Inventor: GOLA, Mauro, CH-6925 Gentilino (CH); SOLDATI, Gianni, CH-6968 Sonvico (CH)
(74) Representative: Zardi, Marco
(86) International application number: PCT/EP2014/051253
(87) International publication number: WO 2014/135300

(56) References cited:
- WO-A1-2006/100651
- WO-A1-2011/070052
- WO-A1-2011/071643
- WO-A2-03/089027
- WO-A2-2010/061866
- US-A1- 2011 053 201

## Description

### FIELD OF THE INVENTION

The present invention relates to a system for extraction of cells from a sample of tissue. The system can be used for extraction of stem cells, particularly human mesenchymal stem cells (hMSCs) from adipose tissue.

### STATE OF THE ART

Adult stem cells have an immense potential interest in the pharmaceutical and medical domains. Adipose tissue is a promising source of stem cells suitable for general cell therapy. The extraction of mesenchymal stem cells from adipose tissue has a huge potential in combination with reconstructive surgery. Currently, stem cells derived from adipose tissue have been employed in breast reconstruction after partial mastectomy; further potential uses relate to treatment of a large number of diseases including Parkinson's, Alzheimer's, bone marrow diseases, cardiac and cerebral crises, burns, diabetes, rheumatoid arthritis and others. The adipose tissue is easily available, accounting for at least 10% of body weight of an individual; harvesting adipose tissue from an adult is a simple procedure and is not an ethical issue.

The current technique for extraction of adipose-derived mesenchymal stem cells has been developed mainly for use during reconstruction surgery. The sample is taken and processed during the surgical procedure and extraction requires around 1.5 hours following a sensitive and long protocol. In particular, a problem of the extraction of stem cells from the tissue is that the sample is subjected to several steps (e.g. washing, digestion and others) which require a dedicated room; hence there are several parts that come into contact with the product and must be sterilized before any further use.

In view of the very promising future applications, there is a great interest in collecting and storing of mesenchymal stem cells from human adult adipose tissue, that could be operated e.g. by a stem cell bank. The above referred technique, however, would be unsuitable for a large scale application. Having been developed for application during a surgical procedure, said technique would reveal a number of drawbacks at a larger scale, including: the considerable time required for a single extraction, the need to process the sample immediately after the extraction from the human body, the number of manual operations, failing to meet the common good manufacturing practices and to ensure top-level sterility and cleanliness at large scale. The present invention aims to provide a solution to this problem.

WO 2011/071643 discloses a blood processor comprising a main unit in the form of a centrifuge rotor, and a disposable set of bags. A bag set comprises a flexible primary separation bag and component bags connected by flexible tubes. The bag set is fitted on the main unit by accommodating the bags in containers thereof; the main unit comprises pinch valves to obstruct or release the connection flexible tubes of the bag set, and further comprises a bag squeezing system to induce transfer of components to/from the bags. The squeezing system comprises a flexible diagram secured to each of the bag containers, and a hydraulic circuit powered by a pumping station. This blood processor, however, has a drawback in that the fluid transfer via pinch valves and squeezing system is unable to provide the high precision which is required for harvesting the mesenchymal stem cells from adipose tissue. For example, the precision may be affected by unforeseeable factors including a deviation from standard performance of the hydraulic circuit, e.g. due to wear or degradation of the gaskets or of the liquid. Also the flow pattern caused by squeezing the bag is not precise. Another drawback is the need of a hydraulic circuit in the main unit.

A description of other prior art blood processing system can be found in WO 2010/061866 and WO 03/082027.

WO 2006/100651 discloses an integrated system for collecting, processing and transplanting cell subsets, including adult stem cells.

### Summary of the invention

The goal of the present invention is to provide a medical device for performing a fully automatic and reliable extraction of stem cells from a sample of tissue, and particularly of mesenchymal stem cells from adipose tissue. The invention aims to provide a sterile, fully-closed processing of the sample of tissue, and a significant reduction of processing time and cost. A further aim of the invention is to provide simultaneous processing of multiple samples with a single machine.

These goals are achieved with a system according to independent claim 1. The system includes basically a main device and a disposable kit for use with said main device. An important aspect of the invention is also a disposable kit according to independent claim 20.

Some preferred features are listed in the dependent claims.

The main device is configured to execute at least one preset program, which corresponds to a protocol for the desired extraction of cells from the sample.

The basic idea underlying the invention is that the disposable kit shall receive the sample of tissue and any reagents or solutions involved in the extraction process. Hence, only the disposable kit is in contact with the tissue and intermediate products during the process. To this purpose, the disposable kit comprises essentially a work chamber for receiving a sample of tissue to be processed, and at least one additional receptacle. In some embodiments the receptacles are in the form of plastic bags. The disposable kit is preferably shipped empty and under vacuum, but some embodiments may provide a disposable kit with some or all of the solution bags pre-filled with a necessary reagent.

The disposable kit comprises fluid transfer means which are operable for transferring an amount of fluid from/to the work chamber and from/to any of said receptacles. For example, said fluid transfer means make it possible to load or discharge a washing solution or a digestion enzyme or any reagent or intermediate product to/from the main work chamber, in accordance with the program executed by the main device.

Said fluid transfer means are controlled by suitable interface means between the main device and the disposable kit.

Preferably, the interface means directly associated with the disposable kit are of a mechanical nature, such as rotary joints, in order to avoid electric parts in the disposable kit and reduce cost and complication.

Said fluid transfer means include a pumping system and a valve system. The term of pumping system denotes a system which is adapted to discharge or suction of an amount of a process fluid to/from the working chamber and to/from the additional receptacle or one of the additional receptacles.

For example, in a preferred embodiment, the fluid transfer means of the disposable kit comprise a piston slidable inside the work chamber and a selection valve. Said piston acts as a syringe to load or discharge an amount of fluid to/from the work chamber, whilst the selection valve provides a selective fluid communication between the work chamber and one of the receptacles which are part of the disposable kit.

More preferably, the disposable kit has a first rotary joint and a second rotary joint which, in use, are connected to respective first motor means and second motor means for actuation of said piston and said valve. Said motor means are preferably servo-motor encoders that may be associated to the main device, or to a part thereof, e.g. to a container where the disposable kit is accommodated, for use with the main device.

Preferably, the piston is moved by means of a screw system comprising a stationary member and a travelling member fixed to the piston, while the valve can be realized with a fixed body and a rotary member.

In a preferred embodiment, the system comprises a container where the disposable kit is encapsulated for use with the main device. Said container is then mounted on a rotating carrier for centrifugation. The container is termed active container because it includes servo-motors for actuation of the aforesaid fluid transfer means of the disposable kit, and heating or cooling means for controlling the temperature of the work chamber or of the solution bags, and suitable power and signal connection with the main device. In a preferred embodiment, the heating means operate according to the Peltier effect.

Preferably, said active container has multiple components including at least a first inner component in direct contact with the disposable kit, and a second outer component to be associated with the main device, e.g. to a rotary carousel for centrifugation. The first component is preferably autoclave-proof. In a preferred embodiment, the first component is a drum and the second component is a capsule which is termed active capsule. More preferably, the active container comprises also a third member, which is a watertight autoclave-proof capsule to receive the drum and the disposable kit.

In a particularly preferred form, the disposable kit comprises a substantially cylindrical body which contains the work chamber, and a plurality of bags which are radially arranged around said cylindrical body. A rotary selection valve is installed on top of the cylindrical body, and conduits connect the inside of the work chamber with each one of the bags, via said selection valve. The work chamber contains a slidable piston which, in cooperation with the selection valve, allows the transfer of controlled amounts of fluid between the chamber and the bags. The work chamber, the bags, the valve and the conduits are all part of the disposable kit which is preferably made of plastic.

The main device can be configured to host a plurality of disposable kits, which are processed simultaneously.

In a preferred embodiment, the main device comprises a rotary carousel and a plurality of the above mentioned active containers are mounted on said rotary carousel. The carousel, when mounted inside the main device, can rotate around a main axis of centrifugation, and each of the active containers can rotate relative to the carousel around a tilting axis. By means of this tilting degree of freedom, the axis of the main chamber can be oriented relative to the axis of centrifugation, in accordance with the desired process step.

As mentioned above, an important aspect of the invention is the disposable kit. In accordance with a preferred embodiment, said disposable kit comprises:
a work chamber for receiving a sample of tissue to be processed, and at least one receptacle,
fluid transfer means which are operable for charging an amount of fluid from said at least one receptacle into the work chamber, or discharging an amount of fluid from the work chamber to said at least one receptacle;
passive control means for actuation of said fluid transfer means when the disposable kit is used in said device.

The control means of the disposable kit are termed passive since no motor is incorporated in the disposable kit, and they are controlled from outside of the disposable kit. This is carried out e.g. by the main device and/or by the above mentioned active container, without any contact with the sample, the intermediate products and the final product (pellet of stem cells).

A further aspect of the invention is a preferred configuration of a valve member of the disposal kit, suitable to promote the accumulation and condensing of the stem-cells containing pellet, according to the attached claims. Said valve member has a chamber designed to promote accumulation of the pellet during transit of a process fluid contained in the working chamber.

Preferably, said valve member has an axial passage and a tangential passage; said accumulation chamber is a tapered chamber coaxially aligned to said axial passage, and said tangential passage is open in the lateral surface of the chamber. More preferably, the end portion of the chamber has a spherical surface, e.g. a portion of sphere or a hemisphere in some embodiments.

The invention has the following advantages. The extraction is performed in a fully automatic way and simultaneously from several tissue samples. Accordingly, the inventive system can process thousands of samples per year. Another advantage is that the sample and the reagents (solutions) are confined in the disposable kit, which is totally isolated from the main device and related components such as the rotary carousel or the active containers. Hence, the overall process ensures safety of the operator, sterility and biologic compatibility. In some embodiments, the disposable kit can be received in a sealed autoclave-proof capsule, so that any item in direct contact with the disposable kit can be sterilized in autoclave before another use, for a maximum degree of sterility. Another advantage of the invention is that the main device follows a preset program and the risk of error is reduced, without the need of highly skilled operators. Hence, the system is particularly suitable for a large scale application.

Another advantage is that the procedure can be optimized thanks to the standardized and fully automatic procedure. According to the invention, the treatment time for extraction of hMSCs from adipose tissue can be around 75 min and the expected result is 10³ - 10⁴ MSC/g. A single system can process thousands of samples per year, being suitable for a large scale collection of stem cells from adipose tissue.

It should be noted that the invention provides that fluid transfer means are part of the disposable set, which is a novel feature over the conventional disposable sets which basically consist of bags possibly connected by flexible tubes. Said fluid transfer means are controlled by the main unit, during execution of a program, via suitable electric and/or mechanical interface. The fact that fluid transfer means like a pumping system and valve system are part of the disposable kit ensures a constant performance over time. Transfer of selected amounts of process fluids or intermediate product is controlled with a higher precision compared to the prior-art bag-squeezing systems. In addition, the main device does not need a hydraulic circuit to squeeze the bags.

A further and noticeable advantage of the invention is given by the accumulation chamber of the valve member, which is specifically designed to collect the stem-cells containing pellet. Hence the stem pellet is easily isolated from the process fluid and can be exported with a high grade of purity as desired.

These and other advantages of the invention will be elucidated with the help of the drawings, which refer to a preferred and non-limitative embodiment.

### Description of figures

Fig. 1 illustrates the main components of a system according to a preferred embodiment of the invention, including a main device and a disposable kit.
Fig. 2 is a perspective view of the disposable kit of the system of Fig. 1.
Fig. 3 is a cut-out of the cylindrical body of the disposable kit of Fig. 2, showing the work chamber inside.
Figs. 4 to 6 show some components of the disposable kit.
Fig. 4A is a cross section of the valve member of the disposable kit, according to the embodiment of Fig. 4.
Fig. 4B is a cross section of the valve member of the disposable kit according to a different embodiment.
Fig. 4C shows a fluid pattern in the valve member.
Figs. 7 and 8 show a drum where the disposable kit can be received.
Fig. 8A shows a preferred embodiment of a heating module.
Figs. 9 and 10 show a sealed capsule where the drum of Figs. 7 and 8, together with the disposable kit of Fig. 2, can be received.
Fig. 11 shows an embodiment of an open-frame active capsule where the sealed capsule of Figs. 9, 10 can be received.
Fig. 12 shows the assembly of open-frame active capsule of Fig. 11 and sealed capsule of Figs. 9, 10.
Fig. 13 is an exploded view of a bottom of the open-frame active capsule of Fig. 11.
Fig. 14 is a carousel that can receive a number of active capsules such as the open-frame active capsule of Fig. 11, and is received in the main device.

### Description of a preferred embodiment

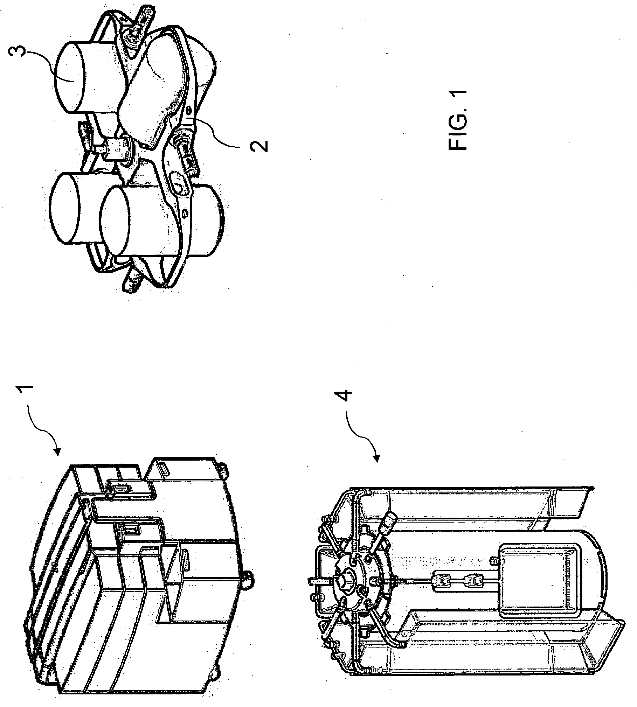

Fig. 1 illustrates a system for the automatic extraction of cells from a sample of tissue. According to a preferred application of the invention, said system is used for performing a protocol for extraction of hMSCs from a sample of human adipose tissue.

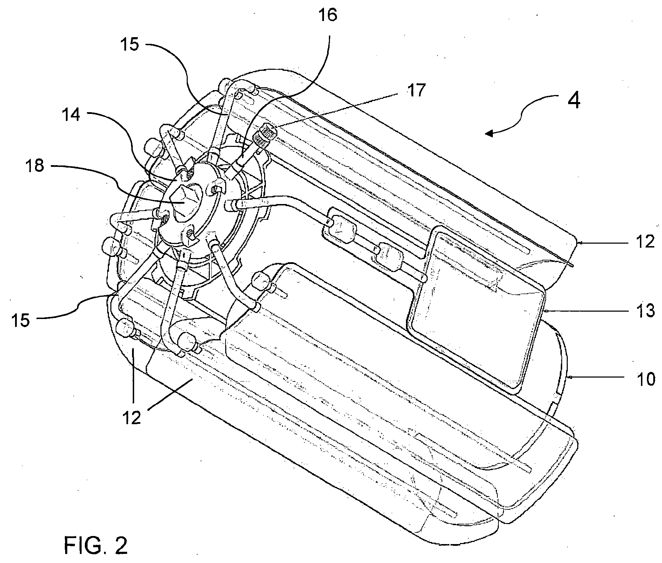

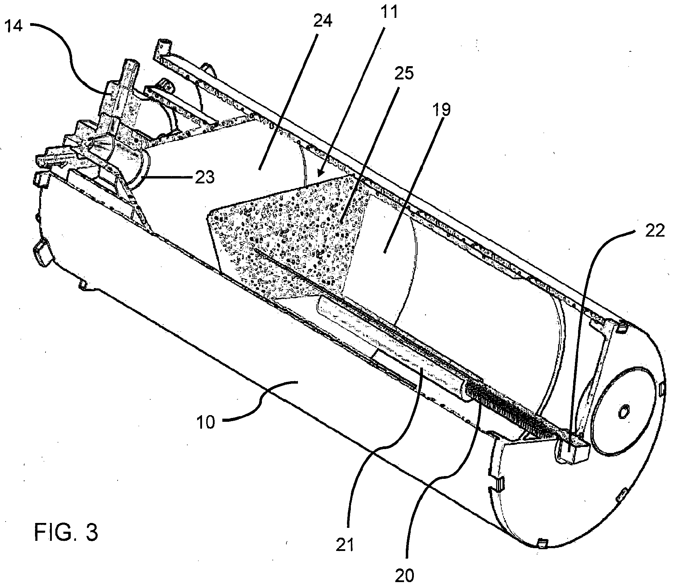

The system comprises a main device 1 including a rotary carousel 2 which carries a plurality of containers 3 Said containers are termed active containers. An important part of the system is a disposable kit 4 where the sample is introduced. Each container 3 hosts a disposable kit 4 comprising a respective adipose tissue sample, so that the system is able to process more than one sample at a time. For example, four samples are processed simultaneously according to the embodiment of Fig. 1. The items in Fig. 1 are not in scale. Referring now to Figs. 2 and 3, the shown embodiment of the disposable kit 4 comprises a cylindrical body 10 delimiting a work chamber 11, a number of solution bags 12 arranged around said body 10 in a radial manner, and a pellet bag 13.

The solution bags 12 and the pellet bag 13 are connected to the inside of the work chamber 11 by means of a valve 14 and respective conduits 15. The valve 14 acts as a collector of several conduits 15 for each solution bag 12 and for the pellet bag 13. The valve 14 is also fitted with an inlet tube 16 and a valve 17 for connection of a syringe and introduction of a sample of tissue in the work chamber 11.

The valve 14 is able to provide a selective communication between the work chamber 11 and one of the solution bags 12 or pellet bag 13, or inlet tube 16, while isolating said chamber 11 from the other parts of the disposable kit. To this purpose, in a preferred embodiment, the valve 14 comprises a rotary member actuated by rotation of a square fitting 18.

Referring to Fig. 3, a sliding piston 19 is arranged inside the working chamber 11 and is operable via a screw system including an axially stationary rod screw 20 and a travelling nut screw 21. The nut screw 21 is firmly fixed to the sliding piston 19, so that a rotation of the rod 20 causes an axial sliding of the piston 19 inside the chamber 11. The rod 20 can be actuated by means of a square fitting 22 accessible from outside of the chamber 11, at the base of the cylindrical body 10.

The chamber 11 has an upper port 23 in communication with the valve 14, for admission of a fluid into the chamber 11 or vice-versa for discharging an amount of fluid from said chamber. Preferably, according to the shown embodiment, the upper region 24 of the chamber 11 is substantially conical, converging to the upper port 23, and more preferably the head 25 of the piston 19 is also conical.

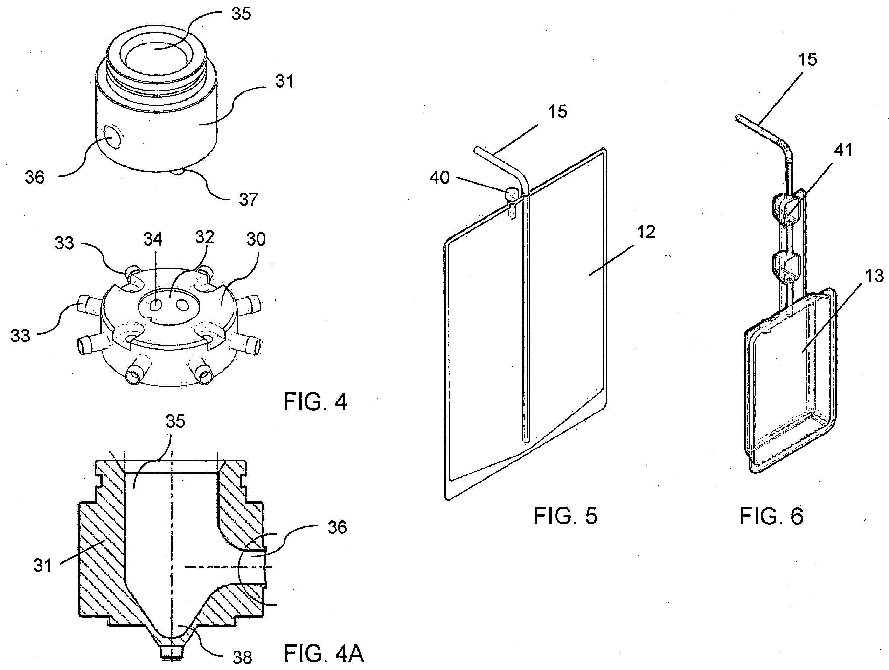

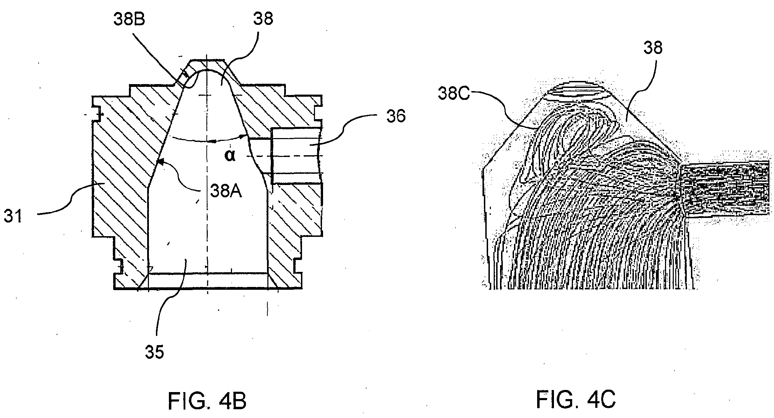

The valve 14 provides a selective communication between the port 23 of the chamber 11 and one of the conduits 15, or the conduit 16 for admission of the sample. The main components of said valve, as shown in Fig. 4, are a valve body 30 and a rotary member 31.

The valve body 30 has an annular shape with a central opening 32 where the rotary member 31 is accommodated. A number of fittings 33, one for each of conduits 15 or 16, depart from the valve body 30 and each fitting 33 is in communication with the central opening 32, via a respective passage 34.

The rotary member 31 has a first port and a second port in communication with each other. When the valve assembly is mounted, said first port is in direct communication with the inlet/outlet port 23 of the work chamber 11, while said second port can be put in a selective communication with one of the passages 34 of the valve body 30, depending on the position of the rotary member 31 relative to said valve body 30. In the shown embodiment, the first port is an axial passage 35 and the second port is a radial hole 36. When the hole 36 faces one of the passages 34, the work chamber 11 is in communication with the corresponding tube fitting 33 and related solution bag 12, pellet bag 13 or the inlet port 16.

The rotary member 31 has also suitable pins 37, or equivalent means, for connection with an actuating member which carry the above mentioned square fitting 18.

A cross section of the rotary member 31 is shown in Fig. 4A. Preferably, as seen in this figure, the axial passage 35 ends with a substantially conical throat 38 for accumulation of the desired stem cell pellet, during the transit of fluid through said valve 14.

Referring also to Fig. 4B, a preferred embodiment of a valve member of the disposable kit, such as the valve rotary member 31, has an axial passage 35 and a tangential passage embodied by the hole 36. Said accumulation chamber 38 is a tapered chamber coaxially aligned to said axial passage 35, and has a lateral surface 38A and a tip surface 38B. The tangential passage of hole 36 is open in the lateral surface 38A. The lateral surface 38A is preferably conical and the tip surface 38B is preferably hemispherical or a portion of a spherical surface. The cone angle of the lateral surface 38A is denoted by α (alpha) in the Fig. 4B, and is preferably between 10° and 30°, more preferably around 20°.

Fig. 4C shows a computational fluid dynamic analysis of the flow entering the radial hole 36. It can be seen that, due to the particular shape and location of the chamber 38, the collection of the heavier and dense pellet which contains the stem-cells is collected in the tip of the chamber 38, due to a region of a strong circulation density denoted by the flow lines 38C and located around the tip of the chamber. This is a considerable advantage of the invention since it avoids that the precious pellet is lost during the intermediate step of the process, when process fluid is expelled from the working chamber 11.

It can be appreciated that the introduction or discharge of fluids to/from the main chamber 11, and to/from any of the bags 12 or pellet bag 13, can be accurately controlled from the outside of the disposable kit 4, and without any direct contact with the sample, by means of the square fittings 18 (rotary valve) and 22 (piston). In use, said fittings 18 and 22 are connected to dedicated motor means such as servo-motor encoders, which will be described hereinbelow, allowing for accurate control of the position of both the valve member 31 and piston 19, in accordance with the various steps of the program executed by the main device.

Some preferred details of the solution bag 12 and pellet bag 13 are shown in Figs. 5 and 6. The solution bag has an inlet cap 40 for introduction of a solution, before the use of the disposable kit 4, in case the bag 12 is shipped empty. The pellet bag 13 has a conduit 15 characterized by at least one small chamber 41 for collection of a sample of the extracted cells, while the cells are delivered from the chamber 11 to said pellet bag 13, at the end of the process.

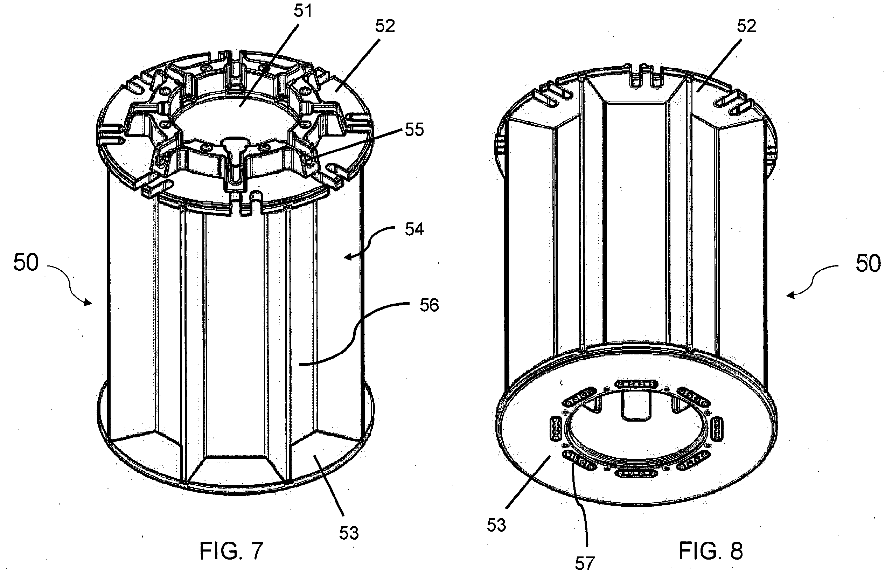

Figs. 7 to 14 illustrate a preferred embodiment of some further components of the system, for use of the disposable kit 4 in the main device 1. Said components form the active container 3 which is schematized in Fig. 1.

Basically, the active container 3 comprises autoclave-proof drum and capsule, in a closer contact with the disposable kit, and an active capsule for mounting on the carousel 2.

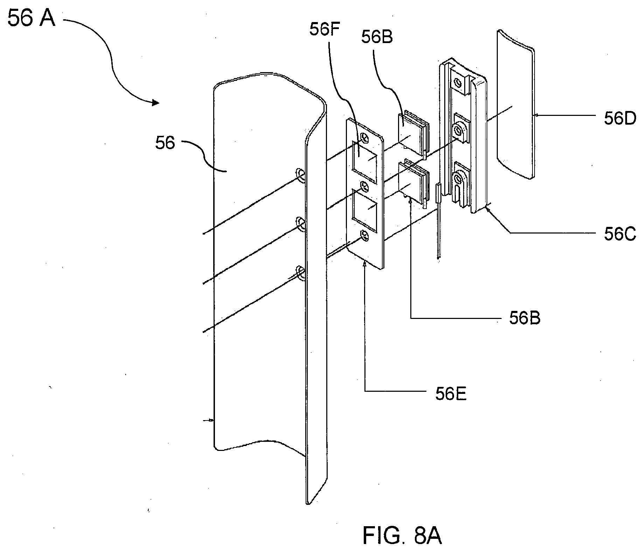

Figs. 7 and 8 illustrate a drum 50 which is configured to receive one of the disposable kits 4. The drum 50 has substantially a cylindrical body with a central passage 51, configured to receive the cylindrical body 10 of the disposable kit 4. The drum 50 comprises also an upper flange 52 and a lower flange 53, and a plurality of seats 54 between the upper and lower flange, for receiving the solution bags 12 and the pellet bag 13. The upper flange 52 includes a number of seats 55 for accommodation of the conduits 15 or 16, so that the drum and disposable kit form a substantially rigid body when assembled together.

Preferably, the drum 50 embodies suitable thermal elements for heating and possibly cooling the work chamber 11 or solution bags 12. Said thermal elements are powered through suitable electric connections of the drum 50, and an advantage of said thermal elements being associated to the drum 50 is that no electric part is provided in the disposable kit 4 and hence cost of the disposable kit is reduced.

Even more preferably, said thermal elements include thermoelectric elements which provide a heating or cooling action by the known Peltier effect. In a preferred embodiment, as shown in the figures, the drum 50 includes a plurality of thermoelectric elements 56 which form a wall of said seats 54, being in contact with the solution bags 12. Said thermal elements 56 are powered by electrical power connections 57 provided on the lower flange 53 (Fig. 8).

Fig. 8A illustrates a preferred embodiment of a thermal element 56 powered by a heating module 56A, including two Peltier-effect heating modules 56B mounted on a support flange 56C and having a back insulating layer 56D and a front insulator 56E. The front insulator has two windows 56F to allow conduction of heat from the heating modules 56B to the element 56.

In some embodiments, the temperature of each of the thermal elements 56 can be controlled independently.

Preferably the drum 50 is autoclave-proof, so that it can be sterilized after use. This is a preferred measure since the drum 50 is in direct contact with the disposable kit.

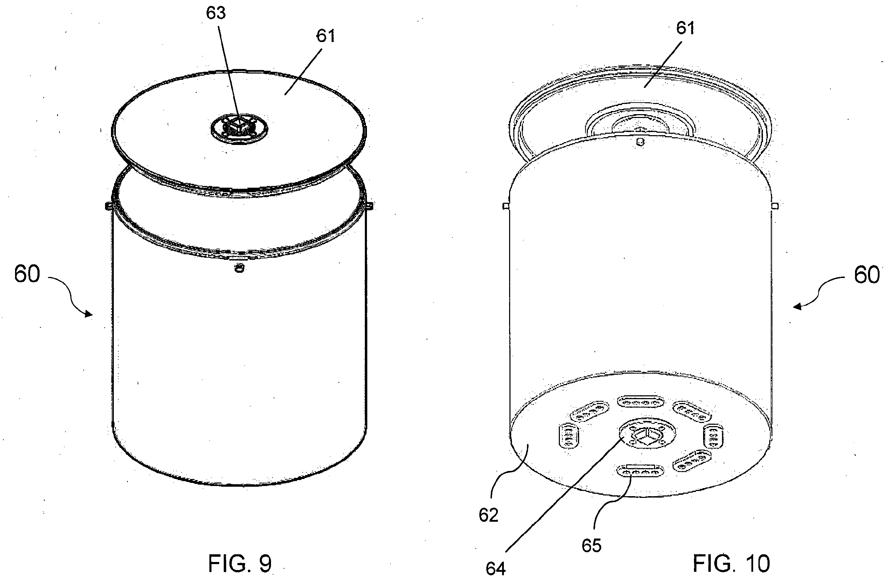

Once the disposable kit 4 is fitted on the drum 50, the assembly of drum and disposable kit is inserted in a watertight, autoclave-proof capsule 60 (Figs. 9, 10) which is another component of the system and, more specifically, of the active container 3.

Said sealed capsule 60 has a cap 61 and a bottom 62 with transmission shafts 63, 64. The termination of said shafts 63, 64 matches the square fittings 18 and 22 of the disposable kit 4, when the kit is inside the capsule 60, so that the valve and piston can be controlled from outside the capsule 60. The bottom 61 has also electric contacts 65 matching the contacts 57 of the drum 50.

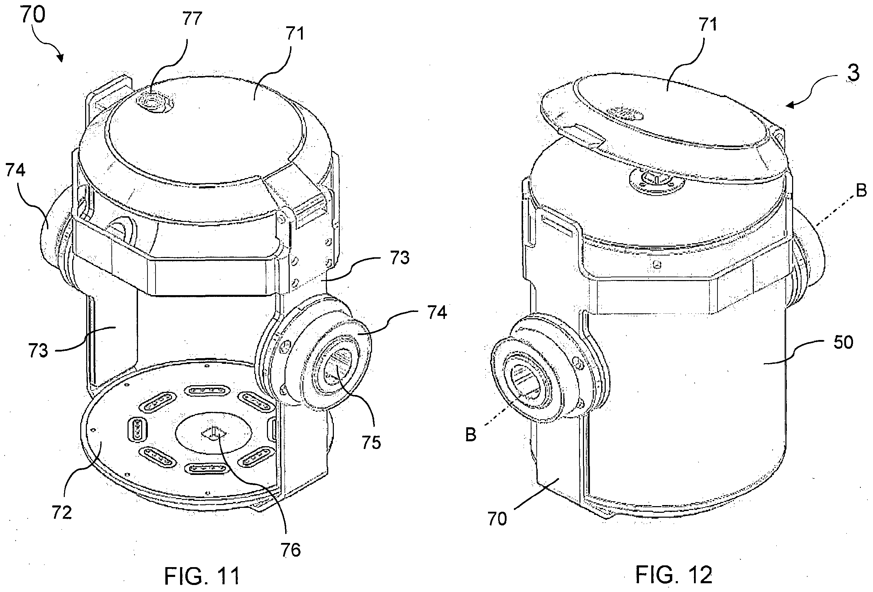

The sealed capsule 60 is received into an active capsule 70 (Fig. 11) to form the active container 3 (Fig. 12) ready for mounting on the rotary carousel 2. Fig. 11 show an open-frame embodiment of said active capsule 70, but an integral-body embodiment is equally possible.

More in detail, the shown embodiment of active capsule 70 comprises an active cap 71 and bottom 72, connected by vertical beams 73 which carry connectors 74. The connectors 74 include preferably a bearing 75 for rotation around an axis B-B (Fig. 12), and further include electric power and signal links.

The cap 71 and bottom 72 house the motors for the actuation of the valve 14 and piston 19, and suitable transmission means to match the transmission shafts 63 or 64, respectively, of the capsule 60. For example, Fig. 11 shows a female connector 76 that match the transmission shaft 64 of bottom of capsule 60. The figure also shows an active cap lock 77 of the cap 71.

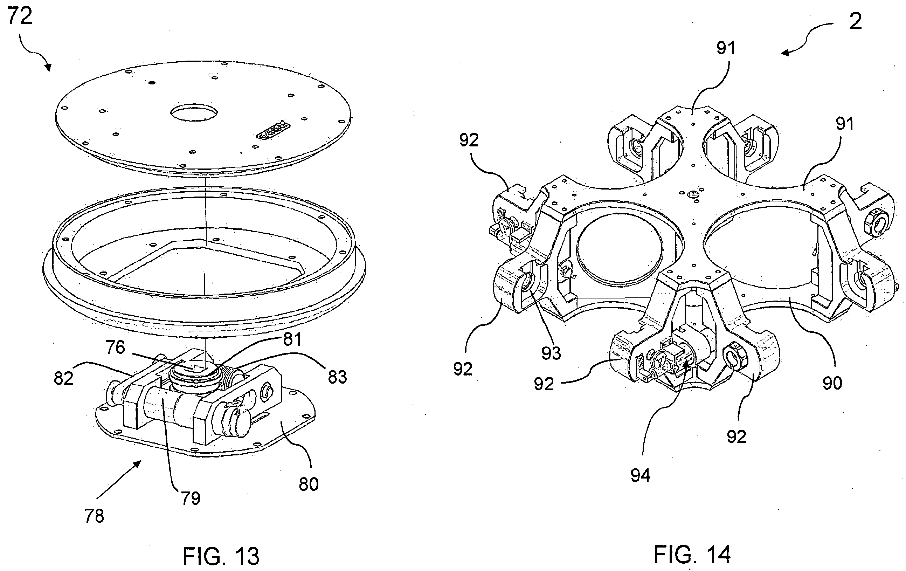

Fig. 13 is an exploded view of a possible embodiment of the bottom 72, showing the motor assembly 78 inside. The motor assembly 78 comprises a servo-motor 79 with a built-in encoder, mounted on a base plate 80 and driving a transmission member 81 via a belt 82 and a worm gear 83. The transmission member 81 carries the female connector 76 for transmission of torque to the shaft 64. Similarly, a second motor assembly is housed in the cap 71, for operation of the valve 14.

As stated before, the capsule 3, or a plurality thereof, is/are mounted onto a rotating carousel 2. Fig. 14 depicts a preferred embodiment of said carousel 2, having a body 90 with four arms 91. Each arm 91 carries a couple flanges 92 arranged at 90°, so that one capsule 3 can be fitted between two flanges of adjoining arms 91, the connectors 74 being received in seats 93 of said flanges. Each arm 81 carries also a motor unit 94 (e.g. a servo-motor with encoder) for providing independent rotation of each capsule 3 around the axis between the two flanges, such as axis B-B.

It can be appreciated that the main device 1 is able to perform mechanical and heating/cooling operations on the content of the disposable kit 4, by means of the active capsule 3 and related components.

More in detail, the sliding piston 19 is controlled by the motor assembly 78 in the base of the active capsule 70, via the connector 64 of the sealed capsule 60 and the and the square fitting 22 of the disposable kit 4, whilst the other motor assembly housed in the cap 71 controls the position of the rotary member 31 of the valve 14 via the connector 63 and the square fitting 18.

Accordingly, the device 1 can precisely control the suction of a fluid content into the work chamber 11, from any of the solution bags 12 or from the sample admission conduit 16, while the sample is being introduced in the chamber 11 at the beginning of the process; in the same way the device can control the delivery of a fluid content from the chamber 11 to any of the bags 12 or to the pellet bag 13, when the process is completed. In addition, the device 1 can heat or cool the content of any of the bags 12 by means of the thermoelectric elements of the drum 50, via the related power connections. Rotation of the carousel 2 provides centrifugation of the capsules.

The system of the invention, in a preferred embodiment, operates as follows.

The solution bags 12 are filled with a syringe inserted in the filler cap 40, and a syringe containing the adipose tissue is connected to the inlet valve 17. The adipose tissue is admitted manually or with an automatic procedure carried by the main device 1. After introduction of the sample, the syringe is removed and the charging tube 16 may be welded. Then, the disposable kit 4 mounted all around the drum 50 and inserted in the active capsule 70 is loaded in the main device and the fully automated procedure is started.

### Example

The following is an example of a protocol carried out with the inventive device and disposable kit. The protocol is based on the fact that adipose tissue and hydrophilic fluids spontaneously separate in two phases without need of centrifugation.

The piston 19 of the separation chamber 11 inside the device is used to take in or to expel the solutions used to wash the sample, to dissociate the suctioned fat, or to extract the cells from the dissociated adipose tissue.

The disposable kit is charged with 150 g of adipose tissue. As a first step, 150 ml of PBS/Ca⁺⁺Mg⁺⁺ are loaded from a first solution bag into the chamber 11 for a first washing step done by gentle agitation of the active capsule inside the device. Then the agitation stops and the capsule stand vertically for few minutes to allow the separation of the phases and the lower aqueous phase is discarded by pushing the piston 19. This procedure is repeated twice.

The remaining 150 ml of washed adipose tissue is then treated enzymatically to free the cells in the aqueous phase. To this aim the tissue is digested 30 minutes at 37°C with the appropriate amount of enzyme diluted in 15 ml PBS/Ca⁺⁺Mg⁺⁺ under constant but gentle agitation of the capsule. Enzymatic reaction is stopped by addition of 60 ml of PBS supplemented with 1% human albumin (without Ca⁺⁺ and Mg⁺⁺). 150 ml of the solution in the separation chamber and containing the released cells are expulsed in a bag for further manipulations.

The tissue remaining in the main chamber is washed again with 150 ml of PBS/1% human albumin and the washing solution is again stored in the same bag. The tissue remaining in the main chamber is now expulsed in another bag for waste. The 300 ml of the cells in PBS/human albumin are then aspirated back in the main chamber 11 and centrifuged 5 minutes at 15 °C and 400g. The pellet that forms at the bottom of the main chamber is then washed once again with PBS/human albumin by centrifugation 5 minutes at 400g at 15°C and the pellet is again suspended in 22.5 ml of PBS/1% human albumin (without Ca⁺⁺ and Mg⁺⁺) and is then ready to be expulsed in the final bag (cryo-bag) for cryopreservation.

## Claims

1. A system for extraction of cells from a sample of tissue, comprising a main device (1) and a disposable kit (4) for use with said main device,
**characterized in that**:
said main device (1) is configured to host at least one disposable kit and is configured to execute at least one preset program when loaded with at least one disposable kit;
said disposable kit (4) comprises a work chamber (11) for receiving a sample of tissue to be processed, and at least one further receptacle (12, 13),
said disposable kit comprises at least a pumping system and a valve system (14, 19) which are operable for selective introduction of a predetermined amount of fluid from said receptacle (12) into the work chamber (11), or selective discharge of a predetermined amount of fluid from the work chamber (11) to the receptacle or to one of the receptacles (12, 13) included in the disposable kit (4),
said main device comprises first interface means and said disposable kit comprises second interface means adapted to match with the first interface means of the main device,
said pumping system and valve system (14, 19) of the disposable kit (4) being controllable by the main device (1) during the execution of a preset program, via said interface means between the main device and the disposable kit.

2. A system according to claim 1, said interface means between the main device and a disposable kit (4) including one or more of the following:
mechanical connections; electrical power connections; electrical signal connections; a rotating carousel for centrifugation of the disposable kit.

3. A system according to claim 1 or 2, **characterized in that** said disposable kit (4) comprises a plurality of receptacles (12), and said pumping system and valve system are operable for introducing an amount of fluid from a selected receptacle (12) into the work chamber, or from the work chamber into a selected receptacle (12).

4. A system according to any of the previous claims, the disposable kit (4) including a sliding piston inside the work chamber and a valve member for selective connection between the work chamber and any of the receptacles (12, 13) of the disposable kit (4), and said interface means comprising a first rotary joint for actuation of said sliding piston, and a second rotary joint for actuation of said valve member.

5. A system according to claim 4, **characterized in that** the interface means comprises at least a first servo motor encoder for operation of said sliding piston, and a second servo motor encoder for operation of said valve member.

6. A system according to any of the previous claims, **characterized by** comprising a respective container (3) for each of the disposable kits (4), the disposable kit (4) being accommodated in the active container for the use with the main device.

7. A system according to claim 6, said container (3) being a multicomponent container, including at least a first inner component (50) in direct contact with the disposable kit (4), and a second outer component (70) to be associated with the main device (1).

8. A system according to claim 7, said first component comprising heat exchange means (56) for heating or cooling any of the work chamber (11) or receptacles (12) of the disposable kit.

9. A system according to claim 8, said heat exchange means being thermoelectric means working by Peltier effect.

10. A system according to any of claims 7 to 9, **characterized in that** said disposable kit comprises a substantially cylindrical body (10) which contains the work chamber (11), and a plurality of receptacles (12) which are radially arranged around said cylindrical body, and said first component is a drum (50) having a central passage (51) for accommodation of the cylindrical body (10), and a number of peripheral seats (54) for accommodation of the receptacles (12).

11. A system according to any of claims 7 to 10, **characterized in that** said container (3) comprises a third intermediate component which is a sealed, autoclave-proof capsule (60).

12. A system according to any of claims 7 to 11, **characterized in that** said second outer component is a capsule (70) which includes a first servo-motor encoder and a second servo-motor encoder for operation of the piston and valve member of the disposable kit, said capsule also comprising electric power and signal connections for operation of said servo-motors.

13. A system according to claim 12, **characterized in that** said capsule (70), or a plurality thereof, can be mounted on a rotary carrier of the main device, and each capsule is tiltable relative to said carrier, and the carrier comprises a signal connection with the main device and respective motorized means to control the tilting of the capsule or each of the capsules, in an independent manner and in accordance with the preset program executed by the main device.

14. A system according to claim 12 or 13, the capsule-carrier being a carousel configured to host four of said capsules and four disposable kits.

15. A system according to any of the previous claims, said main device comprising at least one preset program and said preset program including at least a sequence of steps of centrifugation and of transfers of predetermined amounts of fluids to/from said work chamber and said at least one receptacle, said steps being executed by the main device loaded with at least one disposable kit, during the execution of the program and by operating said pumping system and valve system of the disposable kit via said interface means.

16. A system according to claim 15, said at least one preset program including also steps of controlled heating or cooling of at least one of the receptacles of the disposable kit loaded in the main device.

17. A system according to claim 15 or 16, said at least one preset program being adapted for extraction of stem cells from a sample of adipose tissue, and preferably mesenchymal stem cells from human adult adipose tissue.

18. A system according claim 17, said program including steps of: washing, enzymatic digestion, regulated heating, regulated cooling, centrifugation, extraction of a cell pellet ready for cryogenic conservation.

19. The use of a system according to any of the previous claims, for extraction of stem cells from adipose tissue, particularly for extraction of mesenchymal stem cells from human adult adipose tissue.

20. A disposable kit (4) for use with a device (1) for the extraction of cells from a sample of tissue, said disposable kit (4) comprising:
a work chamber (11) for receiving a sample of tissue to be processed, and at least one receptacle,
at least a pumping system and a valve system (14, 19) which are operable for charging an amount of fluid from said at least one receptacle into the work chamber, or discharging an amount of fluid from the work chamber to said at least one receptacle;
said disposable kit also comprising interface means which includes control means for actuation of said pumping system and valve system, when the disposable kit is used in said device (1).

21. A disposable kit according to claim 20, said control means including at least a rotary joint.

22. A disposable kit according to claim 20 or 21, comprising a plurality of said receptacles (12, 13), and comprising a valve member for selective connection between the work chamber and any of the receptacles (12, 13) of the disposable kit (4).

23. A disposable kit according to claim 22, comprising a sliding piston inside said work chamber.

24. A disposable kit according to claim 23, **characterized in that** said valve member is a rotary member, and the disposable kit (4) comprises a screw system for operation of said sliding piston, and said control means comprises at least a first rotary joint for controlling said valve member, and a second rotary joint for controlling said screw system.

25. A disposable kit according to claim 24, said screw system comprising an axially stationary member and a travelling member, the travelling member being integral with the piston, and the stationary member having a termination outside the work chamber which form said second rotary joint.

26. A disposable kit according to any of the previous claims, the work chamber having an upper conical region and the sliding piston having a conical head.

27. A disposable kit according to any of the previous claims, said valve member having a chamber (38) for collection of the stem-cells containing pellet.

28. A disposable kit according to claim 27, said valve member having an axial passage (35) and a tangential passage (36), said pellet collection chamber (38) being a tapered chamber coaxially aligned to said axial passage, and said tangential passage (36) being open in a lateral surface (38A) of said chamber.

29. A disposable kit according to claim 28, said lateral surface (38A) being conical, preferably having a cone angle between 10° and 30° and more preferably around 20°.

30. A disposable kit according to any of claims 20 to 29, comprising a substantially cylindrical body which contains the work chamber, and a plurality of receptacles which are radially arranged around said cylindrical body.

31. A disposable kit according to any of claims 20 to 30, said receptacles of the disposable kit including a bag (13) for receiving the cells extracted from the sample of tissue.

32. A disposable kit according to any of claims 20 to 31, said disposable kit being made of a thermoplastic biocompatible material.

33. A disposable kit according to any of claims 20 to 32, the kit being shipped empty and preferably under vacuum.

34. A disposable kit according to any of claims 20 to 33, for use in a process of extraction of stem cells from adipose tissue.

35. A process of extraction of cells form a sample of tissue, particularly for the extraction of stem cells form adipose tissue, **characterized by** the use of a system or use of a disposable kit according to any of the previous claims.

## Patentansprüche

1. System zum Extrahieren von Zellen aus einer Gewebeprobe, das eine Hauptvorrichtung (1) und ein Einweg-Kit (4) zur Verwendung mit der Hauptvorrichtung aufweist,
**dadurch gekennzeichnet, dass** die Hauptvorrichtung (1) dazu ausgebildet ist, mindestens ein Einweg-Kit aufzunehmen, sowie dazu ausgebildet ist, mindestens ein vorgegebenes Programm auszuführen, wenn sie mit mindestens einem Einweg-Kit bestückt ist;
dass das Einweg-Kit (4) eine Arbeitskammer (11) zum Empfangen einer zu verarbeitenden Gewebeprobe sowie mindestens ein weiteres Behältnis (12, 13) aufweist,
dass das Einweg-Kit mindestens ein Pumpsystem und ein Ventilsystem (14, 19) aufweist, die für ein selektives Einbringen einer vorbestimmten Menge von Fluid aus dem Behältnis (12) in die Arbeitskammer (11) oder zum selektiven Abgeben einer vorbestimmten Menge von Fluid aus der Arbeitskammer (11) in das Behältnis oder in eines der Behältnisse (12, 13) betätigbar sind, das bzw. die in dem Einweg-Kit (4) enthalten sind,
dass die Hauptvorrichtung eine erste Schnittstelleneinrichtung aufweist und das Einweg-Kit eine zweite Schnittstelleneinrichtung aufweist, die zu der ersten Schnittstelleneinrichtung der Hauptvorrichtung passend ausgebildet ist, wobei das Pumpsystem und das Ventilsystem (14, 19) des Einweg-Kits (4) mittels der Hauptvorrichtung (1) während der Ausführung eines vorgegebenen Programms über die Schnittstelleneinrichtungen zwischen der Hauptvorrichtung und dem Einweg-Kit steuerbar sind.

2. System nach Anspruch 1,
wobei die Schnittstelleneinrichtung zwischen der Hauptvorrichtung und einem Einweg-Kit (4) eines oder mehrere von Folgendem aufweist:
mechanische Verbindungen; elektrische Energieverbindungen; elektrische Signalverbindungen; ein sich drehendes Karussell zur Zentrifugierung des Einweg-Kits.

3. System nach Anspruch 1 oder 2,
**dadurch gekennzeichnet, dass** das Einweg-Kit (4) eine Mehrzahl von Behältnissen (12) aufweist und das Pumpsystem und das Ventilsystem betätigbar sind, um eine Menge von Fluid von einem ausgewählten Behältnis (12) in die Arbeitskammer oder von der Arbeitskammer in ein ausgewähltes Behältnis (12) einzubringen.

4. System nach einem der vorhergehenden Ansprüche,
wobei das Einweg-Kit (4) einen Gleitkolben im Inneren der Arbeitskammer sowie ein Ventilelement für eine selektive Verbindung zwischen der Arbeitskammer und einem beliebigen der Behältnisse (12, 13) des Einweg-Kits (4) aufweist, und wobei die Schnittstelleneinrichtung eine erste Drehverbindung zur Betätigung des Gleitkolbens sowie eine zweite Drehverbindung zur Betätigung des Ventilelements aufweist.

5. System nach Anspruch 4,
**dadurch gekennzeichnet, dass** die Schnittstelleneinrichtung mindestens einen ersten Servomotor-Codierer für die Betätigung des Gleitkolbens und einen zweiten Servomotor-Codierer für die Betätigung des Ventilelements aufweist.

6. System nach einem der vorhergehenden Ansprüche,
**dadurch gekennzeichnet, dass** es einen jeweiligen Behälter (3) für jedes der Einweg-Kits (4) aufweist, wobei das Einweg-Kit (4) für die Verwendung mit der Hauptvorrichtung in dem aktiven Behälter untergebracht ist.

7. System nach Anspruch 6,
wobei es sich bei dem Behälter (3) um einen Mehrkomponenten-Behälter handelt, der mindestens eine erste innere Komponente (50) in direktem Kontakt mit dem Einweg-Kit (4) sowie eine zweite äußere Komponente (70) beinhaltet, die der Hauptvorrichtung (1) zuzuordnen ist.

8. System nach Anspruch 7,
wobei die erste Komponente eine Wärmetauschereinrichtung (56) zum Erwärmen oder Kühlen einer beliebigen der Arbeitskammer (11) oder der Behältnisse (12) des Einweg-Kits aufweist.

9. System nach Anspruch 8,
wobei die Wärmetauschereinrichtung eine mittels des Peltier-Effekts arbeitende thermoelektrische Einrichtung ist.

10. System nach einem der Ansprüche 7 bis 9,
**dadurch gekennzeichnet, dass** das Einweg-Kit einen im Wesentlichen zylindrischen Körper (10), der die Arbeitskammer (11) enthält, und eine Mehrzahl von Behältnissen (12) aufweist, die radial um den zylindrischen Körper herum angeordnet sind, und wobei die erste Komponente eine Trommel (50) ist, die eine zentrale Passage (51) zur Aufnahme des zylindrischen Körpers (10) sowie eine Anzahl von peripheren Aufnahmen (54) zur Unterbringung der Behältnisse (12) aufweist.

11. System nach einem der Ansprüche 7 bis 10,
**dadurch gekennzeichnet, dass** der Behälter (3) eine dritte zwischengeordnete Komponente aufweist, bei der es sich um eine dicht verschlossene, Autoclavbeständige Kapsel (60) handelt.

12. System nach einem der Ansprüche 7 bis 11,
**dadurch gekennzeichnet, dass** die zweite äußere Komponente eine Kapsel (70) ist, die einen ersten Servomotor-Codierer und einen zweiten Servomotor-Codierer für die Betätigung des Kolbens und des Ventilelements des Einweg-Kits aufweist, wobei die Kapsel auch elektrische Energie- und Signalverbindungen für den Betrieb der Servomotoren aufweist.

13. System nach Anspruch 12,
**dadurch gekennzeichnet, dass** die Kapsel (70) oder eine Mehrzahl derselben an einem Drehträger der Hauptvorrichtung angebracht werden kann und jede Kapsel relativ zu dem Träger geneigt werden kann, und dass der Träger eine Signalverbindung mit der Hauptvorrichtung sowie jeweilige Motoreinrichtungen aufweist, um das Neigen der Kapsel oder jeder der Kapseln in einer unabhängigen Weise und in Übereinstimmung mit dem vorgegebenen, von der Hauptvorrichtung ausgeführten Programm zu steuern.

14. System nach Anspruch 12 oder 13,
wobei der Kapsel-Träger ein Karussell ist, das zur Aufnahme von vier der Kapseln und vier Einweg-Kits ausgebildet ist.

15. System nach einem der vorhergehenden Ansprüche,
wobei die Hauptvorrichtung mindestens ein vorgegebenes Programm aufweist und das vorgegebene Programm mindestens eine Abfolge von Schritten des Zentrifugierens und der Übertragung von vorbestimmten Mengen von Fluiden zu/von der Arbeitskammer und dem mindestens einen Behältnis beinhaltet,
wobei die Schritte von der mit mindestens einem Einweg-Kit bestückten Hauptvorrichtung während der Ausführung des Programms und unter Betätigung des Pumpsystems und des Ventilsystems des Einweg-Kits über die Schnittstelleneinrichtung ausgeführt werden.

16. System nach Anspruch 15,
wobei das mindestens eine vorgegebene Programm auch Schritte der kontrollierten Erwärmung oder Kühlung von mindestens einem der in die Hauptvorrichtung geladenen Behältnisse des Einweg-Kits beinhaltet.

17. System nach Anspruch 15 oder 16,
wobei das mindestens eine vorgegebene Programm zum Extrahieren von Stammzellen aus einer Probe von Fettgewebe, vorzugsweise von mesenchymalen Stammzellen aus menschlichem adulten Fettgewebe, ausgebildet ist.

18. System nach Anspruch 17,
wobei das Programm die folgenden Schritte aufweist: Waschen, enzymatische Verdauung, reguliertes Erwärmen, reguliertes Kühlen, Zentrifugation, Extraktion eines Zellpellets, das für Tieftemperatur-Konservierung bereit ist.

19. Verwendung eines Systems nach einem der vorhergehenden Ansprüche zum Extrahieren von Stammzellen aus Fettgewebe, insbesondere zum Extrahieren von mesenchymalen Stammzellen aus menschlichem adulten Fettgewebe.

20. Einweg-Kit (4) zur Verwendung mit einer Vorrichtung (1) zum Extrahieren von Zellen aus einer Gewebeprobe, wobei das Einweg-Kit (4) Folgendes aufweist:
eine Arbeitskammer (11) zum Aufnehmen einer zu verarbeitenden Gewebeprobe sowie von mindestens einem Behältnis,
mindestens ein Pumpsystem und ein Ventilsystem (14, 19), die zum Einbringen einer Menge von Fluid aus dem mindestens einen Behältnis in die Arbeitskammer oder zum Abgeben einer Menge von Fluid aus der Arbeitskammer in das mindestens eine Behältnis betätigbar sind;
wobei das Einweg-Kit ferner eine Schnittstelleneinrichtung aufweist, die eine Steuereinrichtung zur Betätigung des Pumpsystems und des Ventilsystems beinhaltet, wenn das Einweg-Kit in der Vorrichtung (1) verwendet wird.

21. Einweg-Kit nach Anspruch 20,
wobei die Steuereinrichtung mindestens eine Drehverbindung aufweist.

22. Einweg-Kit nach Anspruch 20 oder 21,
das eine Mehrzahl der Behältnisse (12, 13) aufweist sowie ein Ventilelement zur selektiven Verbindung zwischen der Arbeitskammer und einem beliebigen der Behältnisse (12, 13) des Einweg-Kits (4) aufweist.

23. Einweg-Kit nach Anspruch 22,
das einen Gleitkolben im Inneren der Arbeitskammer aufweist.

24. Einweg-Kit nach Anspruch 23,
**dadurch gekennzeichnet, dass** es sich bei dem Ventilelement um ein Drehelement handelt, und dass das Einweg-Kit (4) ein Spindelsystem für die Betätigung des Gleitkolbens aufweist und die Steuereinrichtung mindestens eine erste Drehverbindung zum Steuern des Ventilelements und eine zweite Drehverbindung zum Steuern des Spindelsystems aufweist.

25. Einweg-Kit nach Anspruch 24,
wobei das Spindelsystem ein axial stationäres Element und ein Laufelement aufweist, wobei das Laufelement mit dem Kolben einstückig ausgebildet ist, und wobei das stationäre Element einen Abschluss außerhalb der Arbeitskammer aufweist, der die zweite Drehverbindung bildet.

26. Einweg-Kit nach einem der vorhergehenden Ansprüche,
wobei die Arbeitskammer einen oberen konischen Bereich aufweist und der Gleitkolben einen konischen Kopf aufweist.

27. Einweg-Kit nach einem der vorhergehenden Ansprüche,
wobei das Ventilelement eine Kammer (38) zum Sammeln des Stammzellen enthaltenden Pellets aufweist.

28. Einweg-Kit nach Anspruch 27,
wobei das Ventilelement eine axiale Passage (35) und eine tangentiale Passage (36) aufweist, wobei die Pellet-Sammelkammer (38) eine sich verjüngende Kammer ist, die mit der axialen Passage koaxial ausgerichtet ist, und wobei die tangentiale Passage (36) in einer Seitenfläche (38A) der Kammer offen ist.

29. Einweg-Kit nach Anspruch 28,
wobei die Seitenfläche (38A) konisch ist und vorzugsweise einen Konuswinkel zwischen 10° und 30° und besonders bevorzugt um etwa 20° aufweist.

30. Einweg-Kit nach einem der Ansprüche 20 bis 29,
mit einem im Wesentlichen zylindrischen Körper, der die Arbeitskammer enthält, und mit einer Mehrzahl von Behältnissen, die radial um den zylindrischen Körper herum angeordnet.

31. Einweg-Kit nach einem der Ansprüche 20 bis 30,
wobei die Behältnisse des Einweg-Kits einen Beutel (13) zum Aufnehmen der aus der Gewebeprobe extrahierten Zellen aufweisen.

32. Einweg-Kit nach einem der Ansprüche 20 bis 31,
wobei das Einweg-Kit aus einem thermoplastischen biokompatiblen Material hergestellt ist.

33. Einweg-Kit nach einem der Ansprüche 20 bis 32,
wobei das Kit leer und vorzugsweise unter Vakuum ausgeliefert wird.

34. Einweg-Kit nach einem der Ansprüche 20 bis 33 zur Verwendung in einem Verfahren zum Extrahieren von Stammzellen aus Fettgewebe.

35. Verfahren zum Extrahieren von Zellen aus einer Gewebeprobe, insbesondere zum Extrahieren von Stammzellen aus Fettgewebe,
**gekennzeichnet durch** die Verwendung eines Systems oder die Verwendung eines Einweg-Kits nach einem der vorhergehenden Ansprüche.

## Revendications

1. Système d'extraction de cellules dans un échantillon de tissu, comportant un dispositif (1) principal et un nécessaire (4) jetable destiné à être utilisé avec ledit dispositif principal,
**caractérisé en ce que** :
ledit dispositif (1) principal est configuré pour accueillir au moins un nécessaire jetable et est configuré pour exécuter au moins un programme préétabli lorsqu'il est chargé d'au moins un nécessaire jetable ;
ledit nécessaire (4) jetable comporte un compartiment (11) de travail destiné à recevoir un échantillon de tissu à traiter, et au moins un autre réceptacle (12, 13),
ledit nécessaire jetable comporte au moins un système de pompage et un système (14, 19) de soupape qui peuvent fonctionner pour une introduction sélective d'une quantité prédéterminée de fluide dudit réceptacle (12) dans le compartiment (11) de travail, ou pour une évacuation sélective d'une quantité prédéterminée de fluide du compartiment (11) de travail au réceptacle (12, 13) ou à un des réceptacles compris dans le nécessaire jetable (4), ledit dispositif principal comporte un premier moyen d'interface et ledit nécessaire jetable comporte un second moyen d'interface conçu pour correspondre audit premier moyen d'interface du dispositif principal,
lesdits système de pompage et système (14, 19) de soupape du nécessaire (4) jetable pouvant être commandés par le dispositif (1) principal durant l'exécution d'un programme préétabli, par l'intermédiaire dudit moyen d'interface entre le dispositif principal et le nécessaire jetable.

2. Système selon la revendication 1, ledit moyen d'interface entre le dispositif principal et un nécessaire (4) jetable comprenant un ou plusieurs des suivants : des liaisons mécaniques ; des liaisons d'alimentation électrique ; des liaisons de signaux électriques ; un carrousel rotatif destiné à la centrifugation du nécessaire jetable.

3. Système selon la revendication 1 ou 2, **caractérisé en ce que** ledit nécessaire (4) jetable comporte une pluralité de réceptacles (12), et lesdits système de pompage et système de soupape peuvent fonctionner pour introduire une quantité de fluide d'un réceptacle (12) sélectionné dans le compartiment de travail, ou du compartiment de travail dans un réceptacle (12) sélectionné.

4. Système selon l'une quelconque des revendications précédentes, le nécessaire (4) jetable comprenant un piston coulissant à l'intérieur du compartiment de travail et un moyen de valve destiné à une liaison sélective entre le compartiment de travail et l'un quelconque des réceptacles (12, 13) du nécessaire (4) jetable, et ledit moyen d'interface comportant un premier joint rotatif pour l'actionnement dudit piston coulissant, et un second joint rotatif pour l'actionnement dudit élément de valve.

5. Système selon la revendication 4, **caractérisé en ce que** le moyen d'interface comporte au moins un premier codeur de servomoteur pour le fonctionnement dudit piston coulissant, et un second codeur de servomoteur pour le fonctionnement dudit élément de soupape.

6. Système selon l'une quelconque des revendications précédentes, **caractérisé en ce qu'**il comporte un contenant (3) respectif pour chacun des nécessaires (4) jetables, le nécessaire (4) jetable étant logé dans le contenant actif pour être utilisé avec le dispositif principal.

7. Système selon la revendication 6, ledit contenant (3) étant un contenant à composantes multiples, comprenant au moins une première composante (50) interne en contact direct avec le nécessaire (4) jetable, et une seconde composante (70) externe à associer au dispositif (1) principal.

8. Système selon la revendication 7, ladite première composante comportant un moyen (56) d'échange de chaleur destiné à chauffer ou à refroidir un quelconque du compartiment (11) de travail ou des réceptacles (12) du nécessaire jetable.

9. Système selon la revendication 8, ledit moyen d'échange de chaleur étant un moyen thermoélectrique fonctionnant par effet Peltier.

10. Système selon l'une quelconque des revendications 7 à 9, **caractérisé en ce que** ledit nécessaire jetable comporte un corps (10) sensiblement cylindrique qui contient le compartiment (11) de travail, et une pluralité de réceptacles (12) qui sont agencés radialement autour dudit corps cylindrique, et ladite première composante est un tambour (50) présentant un passage (51) central destiné à loger le corps (10) cylindrique, et un nombre de sièges (54) périphériques destinés à loger les réceptacles (12).

11. Système selon l'une quelconque des revendications 7 à 10, **caractérisé en ce que** le contenant (3) comporte une troisième composante intermédiaire qui est une capsule (60) scellée résistante à l'autoclave.

12. Système selon l'une quelconque des revendications 7 à 11, **caractérisé en ce que** la seconde composante externe est une capsule (70) qui comprend un premier codeur de servomoteur et un second codeur de servomoteur destinés à faire fonctionner le piston et l'élément de soupape du nécessaire jetable, ladite capsule comportant aussi des liaisons d'alimentation électrique et de signaux destinées à faire fonctionner lesdits servomoteurs.

13. Système selon la revendication 12, **caractérisé en ce que** ladite capsule (70), ou une pluralité de celles-ci, peut être montée sur un support rotatif du dispositif principal, et chaque capsule peut être inclinée par rapport audit support, et le support comporte une liaison de signaux avec le dispositif principal et des moyens motorisés respectifs pour commander l'inclinaison de la capsule ou de chacune des capsules, de manière indépendante et selon le programme préétabli exécuté par le dispositif principal.

14. Système selon la revendication 12 ou 13, le support de capsule étant un carrousel configuré pour accueillir quatre desdites capsules et quatre nécessaires jetables.

15. Système selon l'une quelconque des revendications précédentes, ledit dispositif principal comportant au moins un programme préétabli et ledit programme préétabli comprenant au moins une séquence d'étapes de centrifugation et de transferts de quantités prédéterminées de fluides vers/de puis ledit compartiment de travail et ledit au moins un réceptacle, lesdites étapes étant exécutées par le dispositif principal-chargé d'au moins un nécessaire jetable, durant l'exécution du programme et en faisant fonctionner lesdits système de pompage et système de soupape du nécessaire jetable par l'intermédiaire dudit moyen d'interface.

16. Système selon la revendication 15, ledit au moins un programme préétabli comprenant aussi des étapes de chauffage ou de refroidissement contrôlé d'au moins un des réceptacles du nécessaire jetable chargé dans le dispositif principal.

17. Système selon la revendication 15 ou 16, ledit au moins un programme préétabli étant conçu pour l'extraction de cellules souches dans un échantillon de tissu adipeux, et de préférence de cellules souches mésenchymateuses dans du tissu adipeux d'un adulte humain.

18. Système selon la revendication 17, ledit programme comprenant les étapes de : lavage, digestion enzymatique, chauffage régulé, refroidissement régulé, centrifugation, extraction d'une boulette de cellules prête à être conservée cryogéniquement.

19. Utilisation d'un système selon l'une quelconque des revendications précédentes, pour l'extraction de cellules souches dans du tissu adipeux, tout particulièrement pour l'extraction de cellules souches mésenchymateuses dans du tissu adipeux d'un adulte humain.

20. Nécessaire (4) jetable destiné à être utilisé avec un dispositif (1) d'extraction de cellules dans un échantillon de tissu, ledit nécessaire (4) jetable comportant :
un compartiment (11) de travail destiné à recevoir un échantillon de tissu à traiter, et au moins un réceptacle,
au moins un système de pompage et un système (14, 19) de soupape qui peuvent fonctionner pour charger une quantité de fluide dudit au moins un réceptacle dans le compartiment de travail, ou pour évacuer une quantité de fluide du compartiment de travail vers ledit au moins un réceptacle ;
ledit nécessaire jetable comportant aussi un moyen d'interface qui comprend un moyen de commande destiné à actionner lesdits système de pompage et système de soupape, lorsque le nécessaire jetable est utilisé dans ledit dispositif (1).

21. Nécessaire jetable selon la revendication 20, ledit moyen de commande comprenant au moins un joint rotatif.

22. Nécessaire jetable selon la revendication 20 ou 21, comportant une pluralité desdits réceptacles (12, 13), et comportant un élément de soupape destiné à une liaison sélective entre le compartiment de travail et un quelconque des réceptacles (12, 13) du nécessaire (4) jetable.

23. Nécessaire jetable selon la revendication 22, comportant un piston coulissant à l'intérieur dudit compartiment de travail.

24. Nécessaire jetable selon la revendication 23, **caractérisé en ce que** ledit élément de soupape est un élément rotatif, et le nécessaire (4) jetable comporte un système de vis pour le fonctionnement dudit piston coulissant, et ledit moyen de commande comporte au moins un premier joint rotatif pour commander ledit élément de soupape, et un second joint rotatif pour commander ledit système de vis.

25. Nécessaire jetable selon la revendication 24, ledit système de vis comportant un élément fixe dans le sens axial et un élément mobile, l'élément mobile faisant partie intégrante du piston, et l'élément fixe ayant une terminaison à l'extérieur du compartiment de travail qui forme ledit second joint rotatif.

26. Nécessaire jetable selon l'une quelconque des revendications précédentes, le compartiment de travail présentant une région supérieure conique et le piston coulissant présentant une tête conique.

27. Nécessaire jetable selon l'une quelconque des revendications précédentes, ledit élément de soupape présentant un compartiment (38) destiné au prélèvement de la boulette contenant les cellules souches.

28. Nécessaire jetable selon la revendication 27, ledit élément de soupape présentant un passage (35) axial et un passage (36) tangentiel, ledit compartiment (38) de prélèvement de boulette étant un compartiment fuselé aligné coaxialement audit passage axial, et ledit passage (36) tangentiel étant ouvert dans une surface (38A) latérale dudit compartiment.

29. Nécessaire jetable selon la revendication 28, la surface (38A) latérale étant conique, de préférence ayant un angle de cône entre 10 et 30° et de manière plus souhaitable autour de 20°.

30. Nécessaire jetable selon l'une quelconque des revendications 20 à 29, comportant un corps sensiblement cylindrique qui contient le compartiment de travail, et une pluralité de réceptacles qui sont agencés radialement autour dudit corps cylindrique.

31. Nécessaire jetable selon l'une quelconque des revendications 20 à 30, lesdits réceptacles du nécessaire jetable comprenant un sac (13) destiné à recevoir les cellules extraites dans l'échantillon de tissu.

32. Nécessaire jetable selon l'une quelconque des revendications 20 à 31, ledit nécessaire jetable étant constitué d'un matériau thermoplastique biocompatible.

33. Nécessaire jetable selon l'une quelconque des revendications 20 à 32, le nécessaire étant expédié vide et de préférence sous vide.

34. Nécessaire jetable selon l'une quelconque des revendications 20 à 33, destiné à être utilisé dans un procédé d'extraction de cellules souches dans du tissu adipeux.

35. Procédé d'extraction de cellules dans un échantillon de tissu, en particulier destiné à l'extraction de cellules souches dans du tissu adipeux, **caractérisé par** l'utilisation d'un système ou l'utilisation d'un nécessaire jetable selon l'une quelconque des revendications précédentes.
